# EUROPÄISCHE PATENTSCHRIFT

(11) **EP 0 588 345 B1**
(45) Veröffentlichungstag und Bekanntmachung des Hinweises auf die Patenterteilung: **25.08.1999**
(21) Anmeldenummer: 93114937.1
(22) Anmeldetag: 16.09.1993
(51) Int. Cl.: A61L 2/06

(54) **Dampfsterilisator mit integriertem Dampferzeuger**
Steam sterilizer with integrated steam generator
Stérilisateur à vapeur comportant un générateur de vapeur intégré

(30) Priorität: 17.09.1992 DE 4231204
(43) Veröffentlichungstag der Anmeldung: 23.03.1994
(73) Patentinhaber: C. STIEFENHOFER GmbH, D-86971 Peiting (DE)
(72) Erfinder: Kammermeier, Bernhard, Dipl.-Ing., D-82396 Pähl (DE)
(74) Vertreter: Schaumburg, Karl-Heinz

(56) Entgegenhaltungen:
- WO-A-92/03170

## Beschreibung

Die Erfindung betrifft eine Dampferzeugungsvorrichtung für einen Dampfsterilisator gemäß dem Oberbegriff des Anspruchs 1. Ein solcher Dampfsterilisator ist beispielsweise aus der WO92/03170 bekannt.

Bei vielen technischen Anwendungen, insbesondere aber bei einem Dampsterilisationsprozeß wirkt sich ein hoher Gehalt an Inertgasen im Dampf nachteilig aus. Die Inertgase werden mit dem Speisewasser in den Dampferzeuger eingeschleppt. Sie können durch Entgasung des Nachspeisewassers wesentlich reduziert werden.

Ein bewährtes und häufig angewandtes Verfahren zur Speisewasserentgasung ist das thermische. Dabei wird das Speisewasser vor der Einspeisung auf Siedetemperatur gebracht und die Inertgase werden zusammen mit Dampfschwaden abgekocht.

Probleme entstehen bei der Kesseleinspeisung dieses heißen Nachspeisewassers mittels Druckerhöhungspumpen, da diese häufig im Grenzbereich ihrer zulässigen Betriebsbedingungen arbeiten müssen. Bei Förderung von heißem Wasser wird bei ungünstigen Einbauverhältnisse z. B. der erforderliche NPSH-Wert unterschritten und es kommt zu Kavitationsschäden. Vorzeitige Leistungsminderung und Zerstörung der Pumpe sind die Folgen.

Weitere Gründe sprechen gegen die Verwendung einer Druckerhöhungspumpe. Wenn diese Pumpe aus Messing (Cu-Zn-Legierung) gefertigt wird, geht Zink in Lösung und belastet das Nachspeisewasser und damit den Sterilisationsprozeß. Wenn die Pumpe aus Edelstahl (z.B. Cr-Ni-Mo-Legierung) gefertigt wird, erhöhen sich deren Herstellungskosten und damit der Kaufpreis beträchtlich.

Der Erfindung liegt die Aufgabe zugrunde bei einer Dampferzeugungsvorrichtung der eingangs genannten Art eine Entgasung des Speisewassers zu ermöglichen.

Diese Aufgabe wird durch die im kennzeichnenden Teil des Anspruchs 1 angegebenen Merkmale gelöst. Der mit der Erfindung erzielte Vorteil besteht darin, daß die Funktionen der Speisewasserentgasung und der Kesselnachspeisung in einer Einheit günstig kombiniert worden sind. Wenn das Nachspeisewasser im Nachspeisebehälter aufgeheizt wird, findet gleichzeitig eine thermische Entgasung statt. Die Inertgase und die Dampfschwaden können über die Entlüftungsleitung abziehen. Da eine teure temperaturbeständige Druckerhöhungspumpe und deren elektrische Ansteuerung (Drehmomentanschluß, eventuell Stern-Dreieck-Umschaltung) entfällt, verringern sich die Anschaffungskosten.

Die Betriebssicherheit der Kesselnachspeisung erhöht sich, da der Verschleiß an den vorgesehenen Stellventilen sicher niedriger ist, als an einer Druckerhöhungspumpe. Außerdem besteht bei Ausfall der bauseitigen Speisewasserversorgung die Gefahr des "Trockenlaufens" und dann der Beschädigung der Druckerhöhungspumpe. Bei der vorliegenden Erfindung kann der Ausfall der bauseitigen Speisewasserversorgung zu keinem Anlagenschaden führen.

Vorteilhafte Ausführungsformen der Erfindung sind in den Unteransprüchen angegeben.

Ein Ausführungsbeispiel der Erfindung ist in der Zeichnung dargestellt und wird im folgenden näher beschrieben:

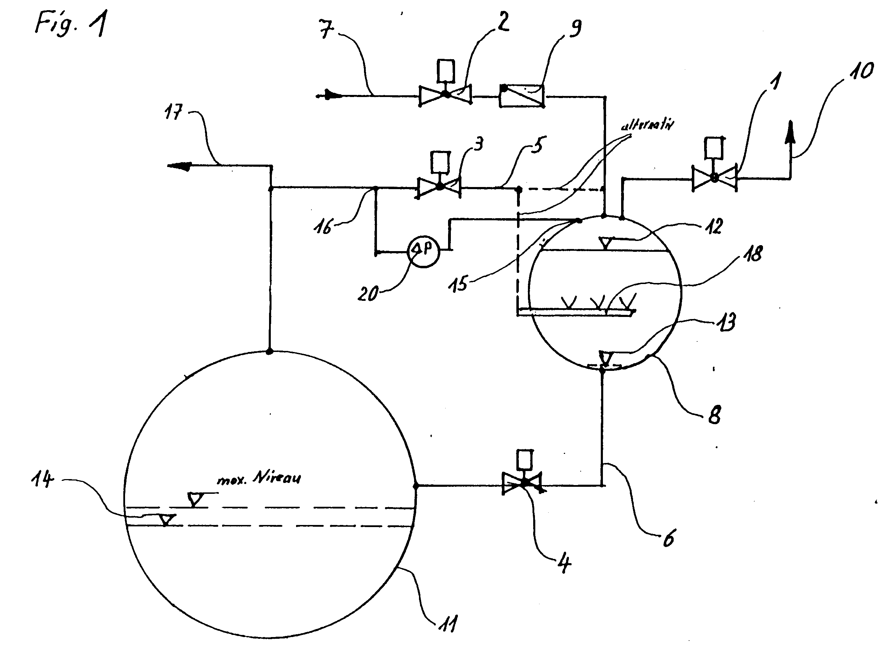

- Fig. 1: zeigt ein Funktionsschema der Erfindung.

In einem Dampferzeuger 11 wird der minimale Wasserstand durch einen Niveauschalter 14 erkannt. Der maximale Wasserstand bei Dampferzeugern stellt sich ein, wenn das gesamte Füllvolumen aus dem Nachspeisebehälter 8 übergeströmt ist.

Im oberen Bereich des Dampferzeugers tritt die Dampfleitung 17 aus. Eine Druckausgleichsleitung 5, in die ein Absperrventil 3 eingebaut ist, tritt aus der Dampfleitung 17 oder direkt aus dem Dampferzeuger aus und führt in den oberen Bereich des Nachspeisebehälters 8. In die Druckausgleichsleitung 5 kann zwischen Absperrventil 3 und Dampferzeuger 11 ein Druckmeßanschluß 16 und zwischen Absperrventil 3 und Nachspeisebehälter 8 ein Druckmeßanschluß 15 für einen Differenzdrucksensor 20 eingebaut sein. Eine Speisewasserzuleitung 7 mit Absperrventil 2 und Rückschlagventil 9 ist in den Nachspeisebehälter 8 in dessen oberen Bereich eingeführt. Ebenfalls in den oberen Bereich des Nachspeisebehälters 8 ist eine Entlüftungsleitung 10 mit einem Absperrventil 1 eingeführt.

Der obere Wasserstand des Nachspeisebehälters 8 wird durch einen Niveauschalter 12 erkannt. Die vollständige Entleerung des Nachspeisebehälters wird durch den Niveauschalter 13 erkannt.

Der Funktionsablauf ohne Entgasung ist unterteilt in die zwei Phasen; Füllen des Nachspeisebehälters 8 und Nachspeisen in den Dampferzeuger 11.

### Füllen des Nachspeisebehälters 8:

Bei offenem Absperrventil 1 und geschlossenen Absperrventilen 2, 3 und 4 ist der Nachspeisebehälter drucklos. Durch Öffnen des Absperrventils 2 kann Speisewasser in den Nachspeisebehälter 8 strömen, bis der Niveauschalter 12 den maximalen Wasserstand im Nachspeisebehälter 8 erkennt und den Steuerimpuls zum Schließen des Absperrventils 2 gibt.

### Nachspeisen in den Dampferzeuger 11:

Der Niveauschalter 14 erkennt den minimalen Wasserstand des Dampferzeugers 11 und gibt den Steuerimpuls für die Nachspeisung. Das Absperrventil 1 wird geschlossen.

Zuerst wird nun das Absperrventil 3 in der Druckausgleichsleitung 5 geöffnet. Da an dem kalten Wasser im Nachspeisebehälter Dampf kondensiert, muß die entsprechende Zeit abgewartet werden, bis das Nachspeisewasser die Temperatur im Dampferzeuger erreicht und sich somit Druckausgleich einstellt.

Der thermische Ausgleich und damit der Druckausgleich kann beschleunigt werden, indem die Druckausgleichsleitung 5 in eine Dampflanze 18 im Nachspeisebehälter 8 mündet oder indem der Nachspeisebehälter 8 unmittelbar an die Wandung des Dampfererzeugers 11 angebaut ist, so daß Wärmeübertragung über die Wandung stattfinden kann.

Anschließend wird das Absperrventil 4 in der Nachspeiseleitung 6 geöffnet. Nachspeisewasser strömt nun aus dem Nachspeisebehälter 8 in den Dampferzeuger 11, bis die Rückschalthystese des Niveauschalters 14 überschritten ist und die vollständige Entleerung des Nachspeisebehälters 8 durch den Niveauschalter 13 gemeldet wird. Anschließend wird der Steuerimpuls zum Schließen der Absperrventile 3 und 4, sowie zum nachfolgenden Öffnen des Absperrventils 1 gegeben. Die abgeblasenen Dampfverluste sind gering.

Der Nachspeisebehälter kann auch in den Dampfraum des Dampferzeugers integriert sein. Die Funktion und Wirkung ist dann, wie beschrieben. Zusätzliche Vorteile sind die Vergrößerung der wärmeübertragenden Flächen, wodurch keine Dampflanze erforderlich ist.

### Funktionsablauf Entgasung

Falls die Kesselnachspeisung mit einer Wasserentgasung kombiniert ist, werden zwischen den beschriebenen Vorgängen "Füllen des Nachspeisebehälters 8" und "Nachspeisen in den Dampferzeuger 11" folgende Funktionen ausgelöst:

Nach Schließen des Absperrventils 2 wird das Absperrventil 3 geöffnet. Das Ventil 1 ist ebenfalls offen. Durch den über die Dampflanze einströmenden Dampf wird das Wasser im Nachspeisebehälter zum Sieden gebracht. Die Dampf- und Gasschwaden ziehen über die Entlüftungsleitung 10 ab. Die Entlüftungsleitung 10 kann in die Atmosphäre führen oder auch an eine Vakuumleitung angeschlossen sein (Vakuumentgasung). Im zweiten Fall wäre die Siedetemperatur dem Vakuum entsprechend niedriger.

Die Dauer des Vorgangs kann zeitgesteuert sein, wobei der Beginn des Siedevorgangs bei Bedarf mit einem Temperatursensor im Wasserraum des Nachspeisebehälters erfaßt werden kann.

## Patentansprüche

1. Dampferzeugungsvorrichtung integriert in einen Dampfsterilisator, mit einem Dampferzeuger (11), einem Nachspeisebehälter (8), der mit dem Dampferzeuger (11) über eine Nachspeiseleitung (6) und eine Druckausgleichsleitung (5) verbunden ist, in denen jeweils ein Absperrventil (4 bzw. 3) eingebaut ist, wobei der Nachspeisebehälter (8) relativ zum Dampferzeuger (11) derart angeordnet ist, daß das niedrigste Füllniveau im Nachspeisebehälter (8) höher liegt als der höchste Füllstand im Dampferzeuger (11), und wobei die Wassernachspeisung vom Nachspeisebehälter in den Dampferzeuger (11) ohne Druckerhöhungspumpe erfolgt, dadurch gekennzeichnet, daß die Druckausgleichsleitung (5) mit einer Dampflanze (18) innerhalb des Nachspeisebehälters (8) verbunden ist und daß der Nachspeisebehälter (8) über eine ein Absperrventil (1) enthaltende Entgasungsleitung (10) mit der Atmosphäre verbunden ist.

2. Dampferzeugungsvorrichtung nach Anspruch 1, dadurch **gekennzeichnet**, daß das Füllvolumen im Nachspeisebehälter (8) größer ist als das Volumen, das der Rückschalthysterese eines Niveauschalters (14) im Dampferzeuger (11) entspricht, zuzüglich der maximal möglichen Verdampfungsmenge während der Nachspeisezeit.

3. Dampferzeugungsvorrichtung nach Anspruch 1 oder 2, dadurch **gekennzeichnet**, daß die Dimensionen der verschiedenen Rohrleitungen und Ventile so gewählt sind, daß das Füllen des Nachspeisebehälters (8), der Druckausgleichsvorgang zwischen dem Dampferzeuger (11) und dem Nachspeisebehälter (8) und das Nachspeisen in dem Dampferzeuger (11) weniger Zeit beanspruchen als das Absinken des Wasserniveaus im Dampferzeuger (11) vom höchsten auf den niedrigsten Wasserstand.

## Claims

1. A steam-generating device integrated in a steam steriliser comprising a steam generator (11), a top-up tank (8) connected to the steam generator (11) via a top-up line (6) and a pressure equalising line (5), in each of which a shut-off valve (4 and 3) is incorporated, wherein the top-up tank (8) is so disposed relative to the steam generator (11) that the lowest filling level in the top-up tank (8) is higher than the highest filling level in the steam generator (11) and wherein top-up water is supplied from the top-up generator to the steam generator (11) without a pressure-increasing pump, characterised in that the pressure equalising line (5) is connected to a steam lance (18) in the top-up tank (8) and in that the top-up tank (8) is connected to atmosphere via a degassing line (10) containing a shut-off valve (1).

2. A steam-generating device according to claim 1, characterised in that the filling volume in the top-up container (8) is greater than the volume corresponding to the delay in switching back a level switch (14) in the steam generator (11) plus the maximum possible amount of evaporation during the topping-up period.

3. A steam-generating device according to claim 1 or 2, characterised in that the various pipelines and valves are so dimensioned that the filling of the top-up tank (8), the process of pressure equalisation between the steam generator (11) and the top-up tank (8), and the topping-up in the steam generator (11) take less time than it takes for the water level in the steam generator (11) to fall from the highest to the lowest level.

## Revendications

1. Dispositif de production de vapeur intégré dans un stérilisateur à vapeur, comprenant un générateur de vapeur (11), un récipient de réalimentation (8) qui est relié au générateur de vapeur (11) par une conduite de réalimentation (6) et par une conduite d'équilibrage de pression (5), dans chacune desquelles est intercalé une vanne d'arrêt (4 ou 3 respectivement), dispositif dans lequel le récipient de réalimentation (8) est disposé, relativement au générateur de vapeur (11), de telle manière que le niveau de remplissage le plus bas dans le récipient de réalimentation (8) soit plus haut que le niveau de remplissage le plus élevé dans le générateur de vapeur (11), et dans lequel la réalimentation en eau depuis le récipient de réalimentation dans le générateur de vapeur (11) s'effectue sans pompe d'élévation de la pression, caractérisé en ce que la conduite (5) d'équilibrage de pression est reliée à une lance à vapeur (18) prévue à l'intérieur du récipient de réalimentation (8), et en ce que le récipient de réalimentation (8) est relié à l'atmosphère par une conduite de dégazage (10) incluant une vanne d'arrêt (1).

2. Dispositif de production de vapeur selon la revendication 1, caractérisé en ce que le volume de remplissage dans le récipient de réalimentation (8) est plus grand que le volume qui correspond à l'hystérésis de retour d'un interrupteur à niveau (14) disposé dans le générateur de vapeur (11), majoré de la quantité maximum possible de vaporisation pendant le temps de réalimentation.

3. Dispositif de production de vapeur selon la revendication 1 ou 2, caractérisé en ce que les dimensions des différentes conduites tubulaires et vannes sont choisies de manière que le remplissage du récipient de réalimentation (8), le processus d'équilibrage de la pression entre le générateur de vapeur (11), et le récipient de réalimentation (8), et la réalimentation dans le générateur de vapeur (11), demandent moins de temps que la chute du niveau d'eau dans le générateur de vapeur (11) depuis le niveau maximum jusqu'au niveau minimum.
